# DEMANDE DE BREVET EUROPEEN

(11) **EP 0 643 962 A1**
(43) Date de publication de la demande: **22.03.1995**
(21) Numéro de dépôt: 94402028.8
(22) Date de dépôt: 12.09.1994
(51) Int. Cl.: A61K 9/00, A61K 31/05, A61K 47/26

(54) **Nouvelle forme galénique de phloroglucinol**

(30) Priorité: 14.09.1993 FR 9310945
(71) Demandeur: LABORATOIRE L. LAFON, F-94701 Maisons Alfort (FR)
(72) Inventeur: Nguyen, Thanh-Tam, F-94450 Limeil Brevannes (FR); Leyder, Joelle, F-94000 Creteil (FR)
(74) Mandataire: Le Guen, Gérard

(57) **Abrégé**

La présente invention concerne un comprimé de phloroglucinol à croquer comprenant de 40 à 150 mg de phloroglucinol et un diluant hydrosoluble.

## Description

La présente invention concerne une nouvelle forme galénique de phloroglucinol.

Le phloroglucinol est un antispasmodique non atropinique.

Ses indications therapeutiques sont les suivantes :
- coliques néphrétiques et douleurs des voies urinaires
- coliques hépatiques et douleurs biliaires
- douleurs intestinales, syndrome dysentériforme
- colites spasmodiques
- dysménorrhées d'origine spasmodique
- dystocies dynamiques.

Le phloroglucinol est commercialisé déjà sous différentes formes galéniques. Ainsi, pour une administration par voie orale, il est proposé sous forme de comprimés dragéifiés (qui se désagrègent en 40 à 50 minutes) et de formes lyophilisées.

La présente invention vise à fournir une nouvelle forme galénique du phloroglucinol pour une administration par voie orale, qui permette :
- une disponibilité rapide du phloroglucinol pour l'organisme,
- une utilisation facile et agréable pour le patient,
- une fabrication simple avec des cadences de production industrielle avantageuse.

La présente invention a pour objet un comprimé de phloroglucinol à croquer, comprenant de 40 à 150 mg de phloroglucinol et un diluant hydrosoluble.

Par diluant hydrosoluble, on désigne un diluant au moins partiellement hydrosoluble.

Comme diluants, on peut utiliser notamment le sorbitol, le mannitol, le lactose, le glucose et plus généralement les polyols et polysaccharides.

Ces diluants peuvent généralement représenter de 500 à 1000 mg par comprimé.

Le comprimé selon l'invention peut contenir en outre des adjuvants classiquement utilisés pour les comprimés tels que édulcorants, aromatisants, délitants et lubrifiants.

Les comprimés selon l'invention peuvent être préparés par mélange des constituants jusqu'à obtenir une composition homogène et compression (compression directe) ou par compression après granulation par voie sèche ou par voie humide.

On donnera ci-après des exemples de comprimés à croquer selon l'invention.

| **EXEMPLE 1** | |
|---|---|
| Phloroglucinol | 62 mg |
| Edulcorant | 0 à 2 mg |
| Arôme | 0 à 5 mg |
| Amidon de maïs (délitant) | 0 à 10 mg |
| Mannitol (diluant) | 500 à 1000 mg |
| Leucine (lubrifiant) | 20 à 50 mg |

| **EXEMPLE 2** | |
|---|---|
| Phloroglucinol | 62 mg |
| Edulcorant | 0 à 2 mg |
| Arôme | 0 à 5 mg |
| Amidon de maïs (délitant) | 0 à 10 mg |
| Mannitol (diluant) | 500 à 1000 mg |
| Talc (lubrifiant) | 0 à 5 mg |
| Stéarate de magnésium (lubrifiant) | 1 à 3 mg |
| Stéaryl fumarate (lubrifiant) | 2 à 10 mg |

| **EXEMPLE 3** | |
|---|---|
| Phloroglucinol | 62 mg |
| Sorbitol (diluant soluble) | 500 à 1000 mg |
| Edulcorant/arôme | 0 à 5 mg |
| Amidon de maïs (délitant) | 0 à 10 mg |
| Leucine (lubrifiant) | 20 à 50 mg |

| **EXEMPLE 4** | |
|---|---|
| Phloroglucinol | 62 mg |
| Sorbitol (diluant soluble) | 500 à 1000 mg |
| Edulcorant/arôme | 0 à 5 mg |
| Amidon de maïs (délitant) | 0 à 10 mg |
| Leucine (lubrifiant) | 20 à 50 mg |
| Talc (lubrifiant) | 0 à 5 mg |
| Stéarate de magnésium (lubrifiant) | 1 à 3 mg |

| **EXEMPLE 5** | |
|---|---|
| Phloroglucinol | 62 mg |
| Sorbitol (diluant soluble) | 500 à 1000 mg |
| Edulcorant/arôme | 0 à 5 mg |
| Amidon de maïs (délitant) | 0 à 10 mg |
| Talc (lubrifiant) | 0 à 5 mg |
| Stéaryl fumarate (lubrifiant) | 2 à 10 mg |

## Revendications

1. Comprimé de phloroglucinol à croquer comprenant de 40 à 150 mg de phloroglucinol et un diluant hydrosoluble.

2. Comprimé selon la revendication 1 comprenant de 500 à 1000 mg de diluant par comprimé.

3. Comprimé selon la revendication 1 ou la revendication 2 dans lequel le diluant est choisi parmi les polyols et les polysaccharides.

4. Comprimé selon la revendication 1 ou la revendication 2 dans lequel le diluant est le sorbitol.

5. Comprimé selon la revendication 1 ou la revendication 2 dans lequel le diluant est le mannitol.

6. Comprimé selon la revendication 1 ou la revendication 2 dans lequel le diluant est le lactose.
